# EUROPEAN PATENT APPLICATION

(11) **EP 4 603 129 A1**
(43) Date of publication of application: **20.08.2025**
(21) Application number: 24777995.2
(22) Date of filing: 26.03.2024
(51) Int. Cl.: A61M 37/00

(54) **SPLIT-TYPE TATTOO NEEDLE CONNECTING STRUCTURE**

(30) Priority: 28.03.2023 CN 202320643645 U
(71) Applicant: Wang, Jian, Suzhou, Jiangsu 215000 (CN); Xia, Tingting, Suzhou, Jiangsu 215000 (CN)
(72) Inventor: Wang, Jian, Suzhou, Jiangsu 215000 (CN); Xia, Tingting, Suzhou, Jiangsu 215000 (CN)
(74) Representative: Herrero & Asociados, S.L.
(86) International application number: PCT/CN2024/083736
(87) International publication number: WO 2024/199207

(57) **Abstract**

The present disclosure provided a connection structure for a separable tattoo needle assembly, the tattoo needle assembly includes a tattoo needle (1) and a needle rod (2), a needle tip portion (3) of the tattoo needle (1) is connected to a bottom of the tattoo needle (1), and a top of the tattoo needle (1) is connected to the needle rod (2). A bottom of the needle rod (2) defines a mounting hole (4) that is coaxially arranged with the needle rod (2), and the mounting hole (4) is configured as a cylindrical structure. The tattoo needle (1) includes a connecting portion (5) and the needle tip portion (3), the connecting portion (5) is configured as a polygonal columnar structure, and the needle tip portion (3) is configured as a polyhedral cone structure with a pointed tip (9) at the bottom of the tattoo needle (1), a top of the needle tip portion (3) is connected with a bottom of the connecting portion (5). An outer diameter of the connecting portion (5) is smaller than an inner diameter of the mounting hole (4), the connecting portion (5) is partially or entirely inserted into the mounting hole (4), an adhesive (6) is arranged between the connecting portion (5) and an inner wall of the mounting hole (4), and the connecting portion (5) and the mounting hole (4) are connected with each other through the adhesive (6). The present disclosure improves the convenience and the efficiency during the assembling of the tattoo needle (1) and the needle rod (2), and the stability and safety of subsequent maintenance is secured.

## Description

### TECHNICAL FIELD

The present invention relates to the field of tattoo needles, and in particular to a connection structure for a separable tattoo needle assembly.

### BACKGROUND

The existing tattoo needles mainly include two types: integrated or detached. For the former, the needle tip portion and the needle rod portion of the integrated tattoo needle are made of the same material and formed as an integrated structure. In the prevailing design of the integrated tattoo needle, the needle tip portion and the needle rod portion are both made of stainless steel, and are seamlessly connected with each other, resembling a sewing needle. For the latter, the needle tip portion and the needle rod portion of the separable tattoo needle are made of different materials, and are connected with each other through a specific structure.

The tattoo needle can be applied to skin by vertical puncturing or horizontal dragging. In order to enhance the efficiency of the operation, the tattoo artists typically do not replace the tattoo needle, using the same tattoo needle for both vertical puncturing and horizontal dragging.

For the separable tattoo needle, the diameter of the tattoo needle may be gradually increased form the needle tip portion to the needle rod portion, to resist the resistance during horizontal dragging, preventing the needle rod from bending. Furthermore, the needle tip portion can made of stiffer materials that is harder than the stainless steel of the conventional integrated tattoo needle, enable the piercing of skin to be performed more efficiency, as a result, the use of separable tattoo needles is becoming more prevalent.

The current connection structures between the needle tip portion and the needle rod portion of the separable tattoo needle mainly include: adhesive bonding connections between two flats and mortise-tenon inserted connections. As exemplified in the patent with application number 2022229143427, titled An Tattoo Needle, Fig. 1 shows an adhesive bonding connection, whereas Fig. 10 and Fig. 11 shows a mortise-tenon inserted connection. However, the above-mentioned connection methods present the following limitations.
1.A high detachment probability: the adhesive bonding connection may be applied between the needle tip portion and the needle rod portion, when the tattoo artists tattooing, and performing a horizontal dragging motion on the skin, the needle may encounter resistance due to the piercing of skin. However, the bonding area between the needle tip portion and the needle rod portion is limited, and the amount of adhesive applied is limited, therefore, the adhesive force generated between the bottom surface of the needle tip portion and the top surface of the needle rod portion is insufficient, which may lead to the needle tip portion separated from the needle rod portion. In particular, the needle tip portion is easily separated from the needle rod portion when tattooing on the hard skin or scarred skin. As a result, the adhesive bonding connection may lead to significant security risks.
2.Adhesive overflow: when applying the adhesive bonding connection between the needle tip portion and the needle rod portion, the bonding area between the needle tip portion and the needle rod portion is limited, during the process of applying the adhesive of the installation, either a slight horizontal deviation of the top surface of the needle rod portion, or an excessive amount of adhesive applied may lead to the overflow of adhesive, and the adhesive overflow may occur on the sidewall of the needle rod portion, thereby increasing the thickness of the needle rod portion, which affects pigment flow and ultimately impacts the tattooing effect.
3.Low assembly efficiency: when the needle tip portion and the needle rod are fixedly connected via a mortise-tenon inserted connection. Since both the needle tip portion and the needle rod are relatively slender, and the cross-sectional areas of the needle tip portion and the needle rod portion are limited, which results in a restricted volume for the mortise-tenon components. Consequently, the resistance generated after engagement of the mortise-tenon components is relatively low, which necessitates high precision in both machining and assembly, leading to a more complex manufacturing and higher costs. Additionally, the alignment process during assembly is challenging, resulting in low efficiency. Furthermore, due to the inserted connection between the mortise- tenon components of the needle tip portion and the needle rod, a sufficient amount of adhesive to enhance the bonding strength may be difficult to provide. As a result, the mortise-tenon inserted connection incurs higher costs, requires a longer time, and is difficult to install and low efficiency.

### SUMMARY OF THE DISCLOSURE

The purpose of the present disclosure is to provide connection structure for a separable tattoo needle assembly, which improves the convenience of installation between the tattooing needle and the needle rod, enhances the bonding strength of the connection, such that the stability and safety of the tattooing process is enhanced.

In order to achieve above purpose, the technical solution adopted in the present disclosure is as follows: a connection structure for a separable tattoo needle assembly includes a tattoo needle and a needle rod, a needle tip portion of the tattoo needle is connected to a bottom of the tattoo needle, and a top of the tattoo needle is connected to the needle rod.

A bottom of the needle rod defines a mounting hole that is coaxially arranged with the needle rod, and the mounting hole is configured as a cylindrical structure, the tattoo needle includes a connecting portion and a needle tip portion, the connecting portion is configured as a polygonal columnar structure, and the needle tip portion is configured as a polyhedral cone structure with a pointed tip at the bottom of the tattoo needle, a top of the needle tip portion is connected with a bottom of the connecting portion.

An outer diameter of the connecting portion is smaller than an inner diameter of the mounting hole, the connecting portion is partially or entirely inserted into the mounting hole, an adhesive is arranged between the connecting portion and the mounting hole, and the connecting portion and an inner wall of the mounting hole are connected with each other through the adhesive.

The needle tip portion is configured as the polyhedral cone structure with the pointed tip at the bottom of the tattoo needle, the top of the needle tip portion is connected with the bottom of the connecting portion, a side edge is formed between two adjacent side surfaces of the needle tip portion, an angle is formed between the side edge and a top surface of the needle tip portion, and the angle is not greater than 90° .

The connecting portion is coaxial with the mounting hole, or a central axis of the connecting portion is parallel to a central axis of the mounting hole.

In the above technical solution, the pointed tip has a longitudinal cross section, formed by cutting the pointed tip with a plane that passes through the pointed tip and is parallel to an axial direction of the needle rod; an angle of the pointed tip in the longitudinal cross section is not greater than 90° .

In the above technical solution, the top of the needle tip portion is fixedly connected with the bottom of the connecting portion, or the needle tip portion and the connecting portion are integrally formed.

In the above technical solution, a reinforcing base is provided to connect the top of the needle tip portion with the bottom of the connecting portion.

In the above technical solution, a pre-embedded pillar is arranged inside the mounting hole to adjust an axial position between the tattoo needle and the needle rod, the pre-embedded pillar is coaxial with the mounting hole, and an outer diameter of the pre-embedded pillar is smaller than or equal to the inner diameter of the mounting hole, the pre-embedded pillar is capable of moving along an axial direction of the mounting hole, the connecting portion is arranged below the pre-embedded pillar.

In the above technical solution, the top of the connecting portion contacts to a bottom surface of the pre-embedded pillar; or the top of the connecting portion is connected with the bottom surface of the pre-embedded pillar by the adhesive.

In the above technical solution, a bottom surface of the connecting portion is flush with a bottom surface of the needle rod, or the bottom surface of the connecting portion is positioned below the bottom surface of the needle rod.

In the above technical solution, the top area of the needle tip portion is smaller than or equal to the bottom area of the connecting portion.

In the above technical solution, each side surface of the connecting portion divides the mounting hole into a plurality of adhesive-containing units, a cross section of each adhesive-containing unit of the plurality of adhesive-containing units is a circular segment, an arc section of the adhesive-containing unit is a minor arc, wherein adjacent adhesive-containing units are either independently arranged or communicate with each other at two endpoints of a chord, which corresponds to the minor arc of the circular segment.

Compared with the exiting art, the present disclosure provided the following advantages due to the application of the technical scheme:
1. In the present disclosure, the needle tip portion is mounted inside the mounting hole via the connecting portion, the connecting portion is configured as a polygonal columnar structure, and the outer diameter of the connecting portion is smaller than the inner diameter of the mounting hole, such that the contact space between the connecting portion and the needle rod is increased, and the contact area for adhesive is expanded. As a result, the connecting strength between the tattoo needle and the needle rod is enhanced, and the stability and the security of the tattooing process is improved.
2. In the present disclosure, the needle tip portion is configured as a polyhedral cone structure with a pointed tip at the bottom of the tattoo needle, an angle is formed between each side edge of the polyhedral cone structure and a top surface of the needle tip portion, and the angle is not greater than 90° . In this way, when tattooing, the tip of the tattoo needle is capable of remaining upright, which prevents fractures of the needle caused by resistance, ensuring safety and stability of the tattooing process. In addition, the angle of the needle tip portion is small, which enhances the sharpness in the skin piercing process, and improves the tattooing effects.
3. In the present disclosure, the tattoo needle and the needle rod is connected with each other by insertion, and the maximum outer diameter of the tattoo needle is smaller than the inner diameter of the mounting hole for the needle rod, and the connection is achieved by using an adhesive, compared with the conventional mortise-tenon inserted connection, the present disclosure enables a rapid insertion, and facilitates a more convenient installation, significantly improves the efficiency during assembling, and reduces installation difficulty.
4. In the present disclosure, a minor arc cavity is formed between the connecting portion and the mounting hole, which not only enhances the bonding strength between the tattoo needle and the needle rod, but also allows excess adhesive to flow along the interior of the mounting hole, preventing adhesive overflow onto the sidewall of the needle rod, which avoids an increase of the thickness of the needle rod caused by adhesive overflow, and the affection of pigment flow is prevented, therefore, the stability and the effects of tattoo is improved.
5. In the present disclosure, a pre-embedded pillar is arranged inside the mounting hole. The pre-embedded pillar is capable of moving along an axial direction of the mounting hole, which allows an adjustment of an axial position between the tattoo needle and the needle rod, and suited to different tattoo needles. In addition, the pre-embedded pillar supports the top of the tattoo needle, ensuring the stability and precision of the tattoo.

In the present disclosure, the bottom of the needle rod defines a cylindrical mounting hole. The cylindrical mounting hole has no directional requirements, and does not require a precise alignment between the connecting portion and the needle rod during assembling, as compared to the conventional mortise-tenon inserted connection. In this way, the difficulty of alignment during assembling is reduced, and assembly efficiency is improved.

In the present disclosure, the connecting portion is configured as a polygonal columnar structure, that is, the connecting portion is non-circular. When the connecting portion and the inner wall of the mounting hole are connected with each other by an adhesive, and before the adhesive solidifies, the non-circular structure of the connecting portion increases the flow resistance of the adhesive, which enables the installation angle of the connecting portion to remain stable, such that the tip of the needle tip portion is fixed, ultimately, the stability and quality of the subsequent tattooing process is ensured.

In the present disclosure, the connection is achieved between the connecting portion and the needle rod. During the assembly of the needle tip portion and the needle rod, the tattoo artists grips the connecting portion, which is configured to have a plurality of side surfaces. In addition, applying force to the outer wall surface of the connecting portion is more convenient, facilitating stable gripping by tattoo artists. In this way, direct gripping of the needle tip portion is prevented, which could lead to damage. Therefore, the efficiency and stability of assembly are improved, and a high assembly qualification rate is achieved.

### BRIEF DESCRIPTION OF THE DRAWINGS

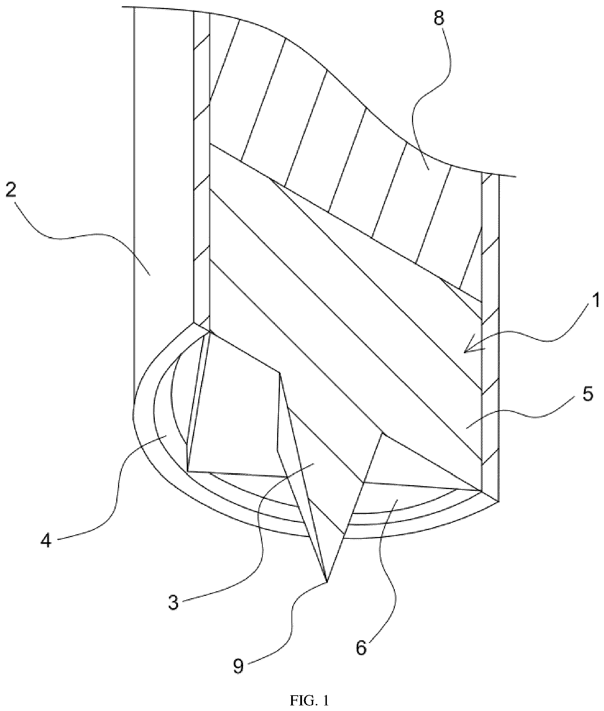

Fig.1 is a structural schematic view according to an embodiment of the present disclosure, shows the state where the top surface of the connecting portion is in contact with the bottom surface of the pre-embedded pillar.

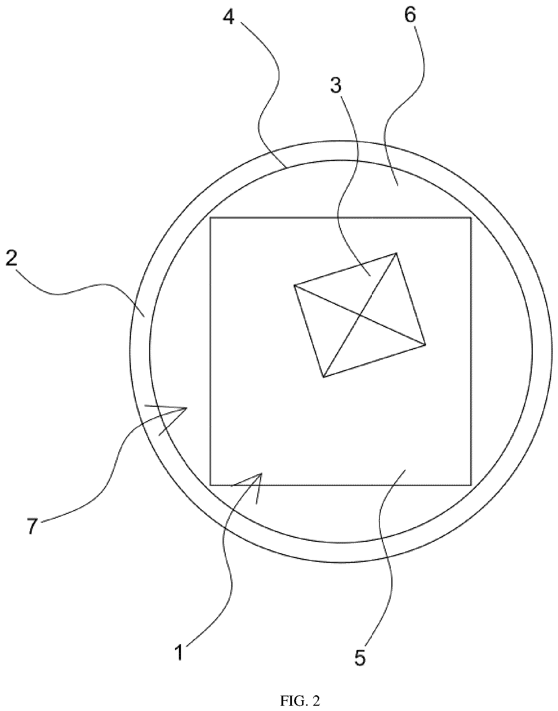

Fig.2 is a structural schematic of an end-view according to an embodiment of the present disclosure, shows a state where the needle tip portion and the connecting portion are not coaxial, and the connecting portion and the mounting hole are either coaxial or substantially coaxial.

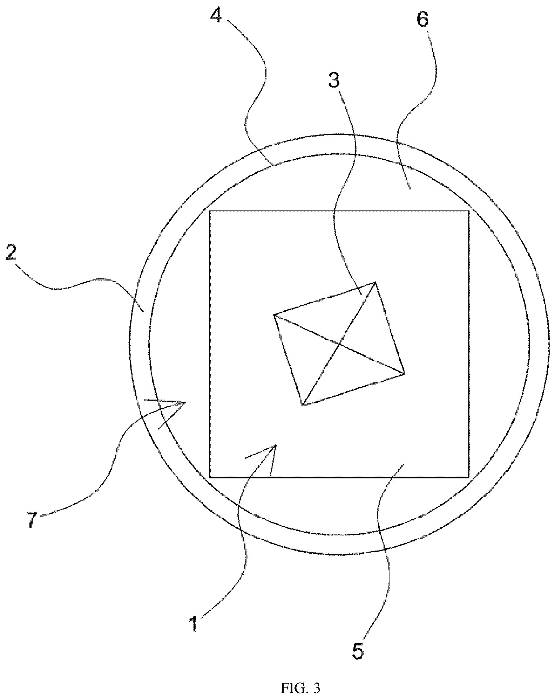

Fig.3 is a structural schematic of an end-view according to an embodiment of the present disclosure, shows a state where the needle tip portion and the connecting portion are coaxial, and the connecting portion and the mounting hole are either coaxial or substantially coaxial.

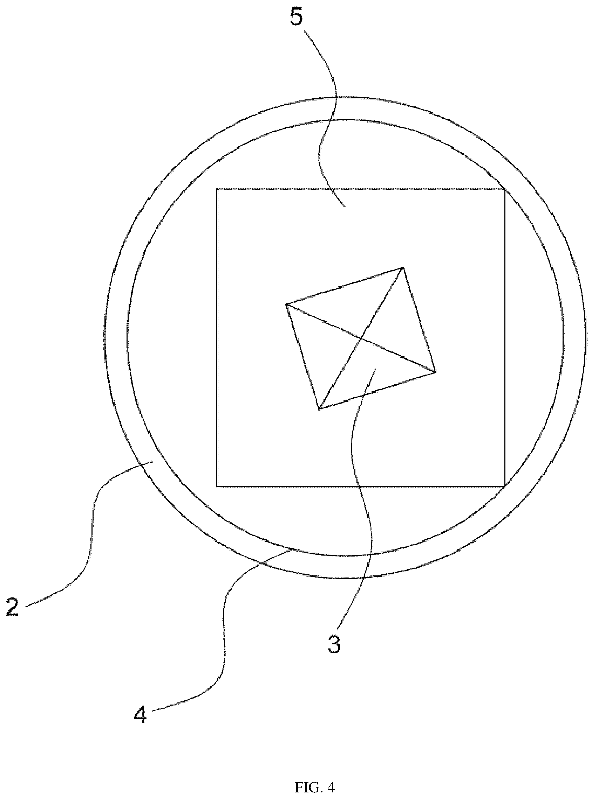

Fig.4 is a structural schematic of an end-view according to an embodiment of the present disclosure, shows a state where the side portion of the connecting portion is in contact with the inner wall of the mounting hole, and the axis of the connecting portion is away from the axis of the mounting hole.

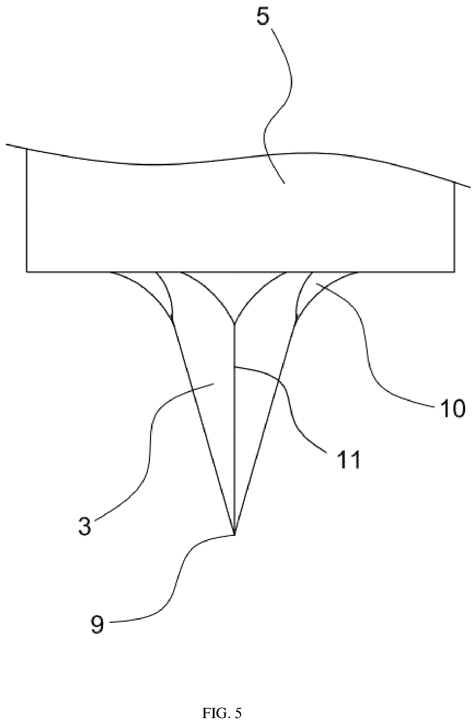

Fig.5 is a partial structural schematic of the tattoo needle according to an embodiment of the present disclosure.

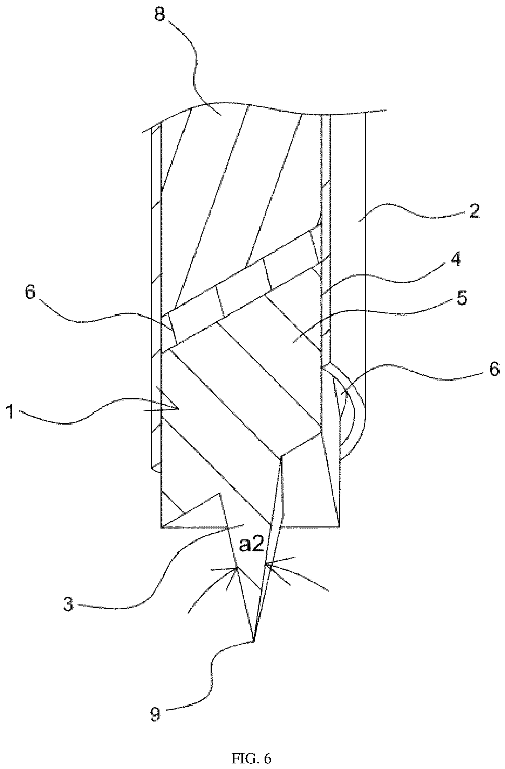

Fig.6 is a structural schematic according to an embodiment of the present disclosure, shows a state where adhesive is applied between the top surface of the connecting portion and the bottom surface of the pre-embedded pillar.

Reference numerals in the drawings: 1, tattoo needle; 2, needle rod; 3, needle tip portion; 4, mounting hole; 5, connecting portion; 6, adhesive; 7, adhesive-containing units; 8, pre-embedded pillar; 9, pointed tip; 10, reinforcing base; 11, side edge.

### DETAILED DESCRIPTION

The present disclosure will be illustrated in the following by referring to the drawings and the embodiments.

Embodiment 1: as shown in Fig.1 to Fig.6, a connection structure for a separable tattoo needle assembly including a tattoo needle 1 and a needle rod 2, a needle tip portion 3 of the tattoo needle 1 provided at the bottom of the tattoo needle, and a top of the tattoo needle 1 connected to the needle rod 2.

A bottom of the needle rod 2 defines a mounting hole 4 that is coaxially arranged with the needle rod 2, and the mounting hole is configured as a cylindrical structure. The tattoo needle includes a connecting portion 5 and the needle tip portion 3, the connecting portion 5 is configured as a polygonal columnar structure, and the needle tip portion 3 is configured as a polyhedral cone structure with a pointed tip at the bottom of the tattoo needle, a top of the needle tip portion 3 is connected with a bottom of the connecting portion 5. It should be understood that the tattoo needle has a polygonal bottom surface, which can be embodied as a triangle, a quadrilateral, a pentagon, or other polygonal shapes. The polyhedral cone structure is formed based on the polygonal bottom surface and includes a plurality of side faces and a corresponding number of side edges 11. For example, when the polygonal bottom surface is a triangle, the polyhedral cone structure includes three side faces and three side edges 11; when the polygonal bottom surface is a quadrilateral, the polyhedral cone structure includes four side faces and four side edges 11; when the polygonal bottom surface is a pentagon, the polyhedral cone structure includes five side faces and five side edges.

An outer diameter of the connecting portion 5 is smaller than an inner diameter of the mounting hole 4, the connecting portion 5 is partially or entirely inserted into the mounting hole 4, an adhesive 6 is arranged between the connecting portion 5 and the mounting hole 4, the connecting portion 5 and an inner wall of the mounting hole 4 are connected with each other through the adhesive 6.

The needle tip portion 3 is configured as the polyhedral cone structure with the pointed tip 9 at the bottom of the tattoo needle 3, the top of the needle tip portion 3 is connected with the bottom of the connecting portion 5, a side edge 11 is formed between two adjacent side surfaces of the needle tip portion 3, an angle is formed between the side edge and a top surface of the needle tip portion 3, and the angle is not greater than 90° .

The connecting portion 5 is coaxial with the mounting hole, or a central axis of the connecting portion is parallel to a central axis of the mounting hole.

In the above embodiment, when the connecting portion is connected with the inner wall of the mounting hole, two types of the cross sections for the connection are provided. In the first type, the connecting portion is arranged at a center of the mounting hole without contacting an inner wall of the mounting hole. As shown in Fig. 2 and Fig. 3, in the case, the adhesive fills the space between the outer wall of the connecting portion and the inner wall of the mounting hole. The connecting portion and the mounting hole are coaxially arranged, or the axis of the connecting portion is closed to the axis of the mounting hole, such that the connecting portion and the needle rod are substantially coaxial. In another type, the connecting portion is offset to one side of the mounting hole, meaning that one side of the connecting portion is in contact with the inner wall of the mounting hole, while a space between the other side of the connecting portion and the mounting hole is larger, in this case, the distance between the axis of the connecting portion and the axis of the mounting hole is larger, leading to a non-coaxial arrangement between the connecting portion and the needle rod, as shown in Fig. 4.

As shown in Fig.1 to Fig. 4, and Fig. 6, each side surface of the connecting portion divides the mounting hole into a plurality of adhesive-containing units, a cross section of each adhesive-containing unit of the plurality of adhesive-containing units is a circular segment, an arc section of the adhesive-containing unit is a minor arc, adjacent adhesive-containing units are either independently arranged or communicate with each other at two endpoints of a chord, which corresponds to the minor arc of the circular segment. Each of the plurality of adhesive-containing units is formed as a minor arc cavity.

In addition, in the present embodiment, the connecting portion is configured as a polygonal columnar structure, in the case, when the connecting portion and the mounting hole are coaxial, each adhesive-containing unit communicates with adjacent adhesive-containing unit, at two endpoints of a chord corresponding to the minor arc of the circular segment, as shown in Fig. 2 and Fig. 3. When the connecting portion and the mounting hole are non-coaxial, and at least one side of the connecting portion is in contact with the inner wall of the mounting hole, some adjacent adhesive accommodating units may be independently arranged, therefore, the minor arc cavity may be a non-standard arc structure, the adhesive is contained within the minor arc cavity to bond the connecting portion and the mounting hole of the needle rod. During the solidifying of the adhesive, a slight vibration is usually applied to the components receiving the adhesive, facilitating the adhesive being evenly distributed. In the case, the slight vibration may be applied to the needle rod, the slight vibration is transmitted through the adhesive inside the mounting hole, causing the connecting portion to vibrate slightly. If the connecting portion is configured as a cylindrical structure, which tends to rotate around the longitudinal axis during the solidifying process of the adhesive, such that bonding strength of the adhesive may be affected. However, in the embodiment of the present disclosure, the connecting portion is configured as a polygonal columnar structure, with multiple side edges formed on the sidewalls. Regardless of whether the connecting portion is in contact with the inner wall of the mounting hole or not, when rotational movement or a tendency to rotate occurs relative to the longitudinal axis, compared the cylindrical structure with smooth, edgeless sidewalls, the polygonal columnar structure can generate greater resistance to impede rotation, therefore, the interference of the adhesive solidifying process is reduced during, and the risk of detachment during use is lowered.

In an embodiment, when assembling the tattoo needle and the needle rod, the mounting hole on the needle rod is positioned facing upward, while the connecting portion of the tattoo needle is positioned facing downward. In this way, when connecting the connecting portion to the mounting hole, the minor arc cavity facilitates the adhesive flowing along the inner wall of the mounting hole, and being away from the interior of the mounting hole. As a result, even with excess adhesive, overflow onto the sidewall of the needle rod is prevented, and the increase of diameter of the needle rod is avoided, such that the affection of the pigment flow is prevented, ensuring the stability and precision of tattooing.

In the present disclosure, a cross section of the mounting hole is circular, while the connecting portion has a polygonal structure. When the connecting portion is inserted into the cylindrical mounting hole, the mounting hole does not have any directional constraint on the connecting portion, which allows the polygonal connecting portion to be inserted at any angle without precise alignment, therefore, the difficulty of calibration is reduced during assembly, while the efficiency and convenience of assembly is improved.

In an embodiment of the present disclosure, an angle formed between the side edge and a top surface of the needle tip portion, is not greater than 90° , in this way, the pointed tip of the needle tip portion is oriented as closely as possible to the axis of the needle tip portion without deviation. The angle formed between the side edge and a top surface of the needle tip portion can be either a right angle or an acute angle, whereas an obtuse angle is not permissible. During the tattooing process, the pointed tip is used for skin piercing, a right angle or an acute angle is suitable for skin piercing, since the resistance of the right angle or the acute angle during skin piercing is smaller. However, if the pointed tip is obtuse, the pointed tip inclines outward, deviating from the axis of the needle tip portion, in this way, when the pointed tip is forced to tilt during skin piercing, the risk of breakage is increased, leading to significant safety risks during tattooing. In addition, if the angle between the side edge and the top surface of the needle tip portion is obtuse, in the installation process (the fixture holds the tattoo needle and the needle rod for assembly), after the assembly of the tattoo needle and the needle rod is completed, as the fixture disengages from the tattoo needle, the needle tip portion may catch onto the fixture (the fixture may drive the needle tip portion to move when disengaging from the tattoo needle), which is not conducive to the installation process.

Therefore, in some embodiments, the needle tip portion is configured as a regular polyhedral pyramid, which facilitates manufacturing and enhances performance. In an embodiment of the present disclosure, the needle tip portion is configured as a regular tetrahedral pyramid, each side edge forms an angle that is not greater than 90° with the bottom surface of the needle tip portion, ensuring the sharpness of the pointed tip, and making the pointed tip suitable for tattooing. Meanwhile, the side edges are inclined, which facilitates the horizontal dragging process, ensuring the quality and effectiveness during horizontal dragging. Furthermore, in the present disclosure, the connecting portion is configured as a polygonal columnar structure, and a regular polygonal columnar structure is optimal. In the present embodiment, the connecting portion is configured as a regular tetrahedral columnar.

As shown in Fig. 6, the pointed tip has a longitudinal cross section, formed by cutting the pointed tip with a plane that passes through the pointed tip and is parallel to an axial direction of the needle rod; an angle of the pointed tip in the longitudinal cross section is not greater than 90° . The angle of the pointed tip is defined as a2, and a2 is less than 90° , that is, the angle of the pointed tip is less than 90° ,which is not formed as an obtuse angle, such that the sharpness is ensured, facilitating the skin piercing.

In an embodiment, the top of the needle tip portion is fixedly connected with the bottom of the connecting portion, or the needle tip portion and the connecting portion are integrally formed.

As shown in Fig. 5, a reinforcing base 10 is provided to connect the top of the needle tip portion and the bottom of the connecting portion with each other. The reinforcing base is arranged at the connection between two side surfaces that are adjacent with each other of the needle tip portion. In the present embodiment, the reinforcing base is served as an arc reinforcing surface, the needle tip portion and the connecting portion are connected with each other, configured as an integral structure. The arc reinforcing surface is also integrally formed with both of the needle tip portion and the connecting portion. By adopting an arc surface as the reinforcing base, the needle tip portion and the connecting portion are connected through the arc surface. Particularly, when a force is applied to perform horizontal dragging, the needle tip portion and the connecting portion are subjected to a radial force, if the connection between the needle tip portion and the connecting portion is not formed as a curved surface, the connection may be broken easily. Therefore, by providing the reinforcing base at the connection between the side edge and the connecting portion, the breakage risk of needle tip is effectively reduced, the stability and safety of tattooing is ensured.

As shown in Fig. 1 and Fig. 6, a pre-embedded pillar is arranged inside the mounting hole to adjust an axial position between the tattoo needle and the needle rod, the pre-embedded pillar is coaxial with the mounting hole, and an outer diameter of the pre-embedded pillar is smaller than or equal to the inner diameter of the mounting hole, the pre-embedded pillar is capable of moving along an axial direction of the mounting hole, the connecting portion is arranged below the pre-embedded pillar.

In the present embodiment, by providing the pre-embedded pillar, the position of the pre-embedded pillar is pre-adjusted, that is, the distance between the bottom surface of the pre-embedded pillar and the bottom surface of the mounting hole is adjusted in advance (the length of the connecting portion that can be installed in the mounting hole is adjusted), then, the tattoo needle is connected to the mounting hole. In this way, tattoo needles of different lengths and needle tip portions with different extension lengths can both be accommodated, such that the applicability of the tattoo needle assembly is enhanced. In addition, according to different application scenarios, such as cases where greater horizontal dragging forces may be applied, the length of the connecting portion installed within the mounting hole can be longer, therefore, the tattoo needle is capable of withstanding greater horizontal dragging forces, preventing the tattoo needle from being detached off from the needle rod, the stability and safety of the tattooing is ensured. In some cases that require the needle tip portion to extend further, the distance between the bottom surface of the pre-embedded pillar and the bottom surface of the mounting hole can be reduced, which allows a longer distance between the bottom of the needle tip portion and the bottom of the needle rod, enabling a greater extension length during the tattooing, and facilitates a wider range of applicability.

The top of the connecting portion contacts the bottom surface of the pre-embedded pillar; alternatively, the top of the connecting portion is bonded to the bottom surface of the embedded pillar through an adhesive.

As shown in Fig.1 and Fig. 6, the bottom surface of the connecting portion is flush with the bottom surface of the needle rod, or the bottom surface of the connecting portion is positioned below the bottom surface of the needle rod.

In the resent disclosure, the bottom of the connecting portion is not arranged inside the mounting hole, but instead positioned below the needle rod. In the present embodiment, the bottom of the connecting portion is flush with the bottom surface of the needle rod.

The top area of the needle tip portion is smaller than or equal to the bottom area of the connecting portion. In some embodiments, the top area of the needle tip portion is smaller than the bottom area of the connecting portion.

In the description of the present disclosure, it should be understood that the terms such as "upper," "lower," "top," "bottom," "inner," and "outer" indicate positional or locational relationships based on the orientations shown in the drawings. These terms are provided solely for the convenience of description and simplification and should not be construed as indicating or implying that the referenced devices or components must have specific orientations or be configured and operated in a particular manner. Therefore, these terms should not be considered as limiting the present invention. In the description of the present invention, the term "multiple" refers to two or more, unless explicitly and specifically defined otherwise.

In the present disclosure, unless otherwise explicitly specified and defined, terms such as "mounting," "connection," "linkage," and "fixation" should be interpreted broadly. For example, these terms may refer to a fixed connection or a separable connection, or an integrated structure. The connection may be mechanical or electrical, direct or indirect through an intermediary medium. For instance, two components may be mechanically abutted or contacted to form a connection, or they may be directly hooked together or mounted through an intermediary medium. Additionally, the connection may refer to internal communication between two components or an interaction between them. Those skilled in the art can understand the specific meanings of these terms in the present disclosure based on the actual circumstances.

## Claims

1. A connection structure for a separable tattoo needle assembly comprising a tattoo needle (1) and a needle rod, wherein a needle tip portion (3) of the tattoo needle (1) is connected to a bottom of the tattoo needle (1), and a top of the tattoo needle (1) is connected to the needle rod (2);
wherein, a bottom of the needle rod (2) defines a mounting hole (4) that is coaxially arranged with the needle rod (2), and the mounting hole (4) is configured as a cylindrical structure, the tattoo needle (1) comprises a connecting portion (5) and the needle tip portion (3), the connecting portion (5) is configured as a polygonal columnar structure, and the needle tip portion (3) is configured as a polyhedral cone structure with a pointed tip (9) at the bottom of the tattoo needle (1), a top of the needle tip portion (3) is connected with a bottom of the connecting portion (5);
wherein, an outer diameter of the connecting portion (5) is smaller than an inner diameter of the mounting hole (4), the connecting portion (5) is partially or entirely inserted into the mounting hole (4), an adhesive (6) is arranged between the connecting portion (5) and the mounting hole (4), the connecting portion (5) and an inner wall of the mounting hole (4) are connected with each other through the adhesive (6);
wherein, the needle tip portion (3) is configured as the polyhedral cone structure with the pointed tip (9) at the bottom of the tattoo needle (1), the top of the needle tip portion (3) is connected with the bottom of the connecting portion (5), a side edge (11) is formed between two adjacent side surfaces of the needle tip portion (3), an angle is formed between the side edge (11) and a top surface of the needle tip portion (3), and the angle is not greater than 90° ;
wherein, the connecting portion (5) is coaxial with the mounting hole (4), or a central axis of the connecting portion (5) is parallel to a central axis of the mounting hole (4).

2. The connection structure for a separable tattoo needle assembly, as recited in claim 1, wherein the pointed tip (9) has a longitudinal cross section, formed by cutting the pointed tip (9) with a plane that passes through the pointed tip (9) and is parallel to an axial direction of the needle rod (2); an angle of the pointed tip (9) in the longitudinal cross section is not greater than 90° .

3. The connection structure for a separable tattoo needle assembly, as recited in claim 1, wherein the top of the needle tip portion (3) is fixedly connected with the bottom of the connecting portion (5), or the needle tip portion (3) and the connecting portion (5) are integrally formed.

4. The connection structure for a separable tattoo needle assembly, as recited in claim 1, wherein a reinforcing base (10) is provided to connect the top of the needle tip portion (3) with the bottom of the connecting portion (5).

5. The connection structure for a separable tattoo needle assembly, as recited in claim 1, wherein a pre-embedded pillar (8) is arranged inside the mounting hole (4) to adjust an axial position between the tattoo needle (1) and the needle rod (2), the pre-embedded pillar (8) is coaxial with the mounting hole (4), and an outer diameter of the pre-embedded pillar (8) is smaller than or equal to the inner diameter of the mounting hole (4), the pre-embedded pillar (8) is capable of moving along an axial direction of the mounting hole (4), the connecting portion (5) is arranged below the pre-embedded pillar (8).

6. The connection structure for a separable tattoo needle assembly, as recited in claim 5, wherein the top of the connecting portion (5) contacts to a bottom surface of the pre-embedded pillar (8); or the top of the connecting portion (5) is connected with the bottom surface of the pre-embedded pillar (8) by the adhesive (6).

7. The connection structure for a separable tattoo needle assembly, as recited in claim 1, wherein a bottom surface of the connecting portion (5) is flush with a bottom surface of the needle rod (2), or the bottom surface of the connecting portion (5) is positioned below the bottom surface of the needle rod (2).

8. The connection structure for a separable tattoo needle assembly, as recited in claim 1, wherein the top area of the needle tip portion (3) is smaller than or equal to the bottom area of the connecting portion (5).

9. The connection structure for a separable tattoo needle assembly, as recited in claim 1, wherein each side surface of the connecting portion (5) divides the mounting hole (4) into a plurality of adhesive-containing units (7), a cross section of each adhesive-containing unit (7)of the plurality of adhesive-containing units (7) is a circular segment, an arc section of the adhesive-containing unit (7) is a minor arc, wherein adjacent adhesive-containing units (7) are either independently arranged or communicate with each other at two endpoints of a chord, which corresponds to the minor arc of the circular segment.
